(19) Europäisches Patentamt
European Patent Office
Office européen des brevets

(11) Publication number: 0 036 292 B1

(12) **EUROPEAN PATENT SPECIFICATION**

(45) Date of publication of patent specification: **16.01.85**

(51) Int. Cl.[4]: **C 07 C 15/00,** C 07 C 2/00, B 01 J 29/28, C 01 B 33/28

(21) Application number: **81301022.0**

(22) Date of filing: **11.03.81**

# (54) Process for the production of aromatic hydrocarbons.

(30) Priority: **15.03.80 GB 8008871**

(43) Date of publication of application: **23.09.81 Bulletin 81/38**

(45) Publication of the grant of the patent: **16.01.85 Bulletin 85/03**

(84) Designated Contracting States: **BE DE FR GB IT NL**

(56) References cited:
**EP-A-0 002 899**
**DE-A-2 817 577**
**US-A-3 972 983**
**US-A-4 108 881**
**US-A-4 139 600**

**The file contains technical information submitted after the application was filed and not included in this specification**

(73) Proprietor: **The British Petroleum Company p.l.c.**
**Britannic House Moor Lane**
**London EC2Y 9BU (GB)**

(72) Inventor: **Ball, William John**
**The British Petroleum Co. Ltd. Chertsey Road**
**Sunbury-on-Thames Middlesex TW16 7LN (GB)**
Inventor: **Stewart, David Gordon**
**The British Petroleum Co. Ltd. Chertsey Road**
**Sunbury-on-Thames Middlesex TW16 7LN (GB)**

(74) Representative: **Harry, John et al**
**c/o The British Petroleum Company plc Patents Division Chertsey Road**
**Sunbury-on-Thames Middlesex TW16 7LN (GB)**

EP 0 036 292 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

The present invention relates to a process for the production of aromatic hydrocarbons by contacting at an elevated temperature and in the vapour phase a $C_2$ to $C_{12}$ aliphatic hydrocarbon feedstock with a catalyst comprising a crystalline aluminosilicate having a silica to alumina molar ratio greater than 12:1 which has been modified by incorporation therein of a metal either by exchange or impregnation.

Crystalline hydrated aluminosilicates, generally referred to as zeolites, may be represented by the empirical formula:

$$M_{2/n}O \,.\, Al_2O_3 \,.\, xSiO_2 \,.\, yH_2O$$

in which n is the valence of M which is generally an element of Group I or II, in particular sodium, potassium, magnesium, calcium, strontium or barium and x is generally equal to or greater than 2. Zeolites have skeletal structures which are made up of three-dimensional networks of $SiO_4$ and $AlO_4$ tetrahedra, corner-linked to each other by shared oxygen atoms. There are no unshared oxygen atoms in the anionic framework so that the ratio of total aluminium and silicon atoms (Al+Si) to oxygen atoms is 1:2 and the negative charges created by the replacement of Si (IV) atoms by Al (III) atoms are neutralised by an electrochemical equivalent of cations M.

Although before the mid-1960s about 100 different types of synthetic zeolites were known it had not been found possible to synthesise zeolites having a silica to alumina molar ratio greater than about 11:1. Thereafter this was achieved by the use in the preparation of one or more quaternary alkylammonium compounds such as tetramethylammonium, tetrapropylammonium and tetrabutylammonium compounds. There resulted a range of crystalline aluminosilicates having a silica to alumina ratio up to 2000:1, high stability, extremely high acidity and the ability to catalyse many kinds of conversion reactions, in particular the conversion of aliphatic compounds into aromatic compounds.

U.S. Patent No. 4,108,881 (Mobil Oil) describes a new form of zeolite ZSM-11 having a formula in terms of mole ratios of oxides in the anhydrous state as follows:

$$(0.5\text{—}10.0)R:(0\text{—}0.5)M_2O:Al_2O_3:xSiO_2$$

wherein M is an alkali metal ion, R is an alkylenediamine having from 7 to 12 carbon atoms or an organic cation derived therefrom, and x is from 10 to 1000, and a characteristic X-ray powder diffraction. The original alkali metal cations (M) are replaceable by cations and preferably cations which render the zeolite catalytically active especially for hydrocarbon conversion, such as hydrogen, rare earth metals, aluminium, metals of Groups IIA, IIIB, IVB, VIB, VIII, IB, IIIA and IVA. The zeolite ZSM-11 is said to be useful in both the unexchanged and the exchanged form in, amongst other conversion reactions, aromatisation of normally gaseous olefins and paraffins and aromatisation of normally liquid low molecular weight paraffins and olefins.

U.S. Patent No. 4139600 (Mobil Oil) describes a synthetic crystalline aluminosilicate zeolite having, as synthesised, a formula in terms of mole ratios of oxides in the anhydrous state as follows:

$$(0.5\text{—}10.0)R:(0\text{—}0.5)M_2O:Al_2O_3:xSiO_2$$

wherein M is an alkali metal ion, R is an alkyldiamine selected from the group consisting of pentanediamine, hexanediamine and combinations thereof or an organic nitrogen-containing cation derived from said alkyldiamine, and x is at least 5, and having a characteristic X-ray powder diffraction pattern. Thereafter the disclosure is similar with respect to replaceable cations and uses to that of U.S.P. 4108881.

Our European patent publication No. 30811, of earlier filing date but published after the filing date of the present application, describes a process for the production of a crystalline aluminosilicate having a silica to alumina molar ratio greater than 12:1 by mixing a source of silica, a source of alumina, a source of alkali metal, water and a source of ammonium ions, and maintaining the mixture at elevated temperature for a period such that crystallisation occurs characterised in that a source of ammonium ions is employed in the absence of an alcohol or alkylene oxide and the source of silica, the source of alumina, the source of alkali metal, water and the source of ammonium ions are mixed in the molar proportions (expressed in the case of the silica and alumina sources in terms of the equivalent moles of the oxide, in the case of the alkali metal source in terms of the equivalent moles of the hydroxide [MOH] and in the case of the source of ammonium ions in terms of free ammonia):

$SiO_2:Al_2O_3$ greater than 12:1
$MOH:Al_2O_3$ in the range from 1:1 to 20:1
$SiO_2:NH_3$ in the range from 1:1 to 200:1, and
$H_2O:MOH$ in the range from 30:1 to 300:1.

The use of the hydrogen-form and the gallium-exchanged form of aluminosilicates so-prepared as catalysts in the conversion of $C_3$ hydrocarbon mixtures to aromatic hydrocarbons is also specifically described in Examples 6 to 9 of the description.

In our British Patent No. 1561590 we disclose that zeolites having a silica to alumina molar ratio of between 20:1 and 70:1 in which gallium is exchanged with the cation M or is impregnated on to the surface of the zeolite and/or into the zeolitic cavities has a surprisingly high

catalytic activity in hydrocarbon conversion processes.

We have now found that certain crystalline aluminosilicates of the type as hereinafter described when modified by incorporation of aluminium, either by exchange or impregnation are, surprisingly, active catalysts for the conversion of $C_2$ to $C_{12}$ aliphatic hydrocarbons to aromatic hydrocarbons.

Accordingly, the present invention provides a process for the production of aromatic hydrocarbons by contacting at an elevated temperature and in the vapour phase a $C_2$ to $C_{12}$ aliphatic hydrocarbon feedstock with a catalyst comprising a crystalline aluminosilicate having a silica to alumina molar ratio greater than 12:1 which has been modified by incorporation therein of a metal either by exchange or impregnation and which is produced by either:

(i) crystallising a mixture containing a source of alumina, a source of silica, a source of alkali metal, water and a mono-, di- or tri-ethanolamine or propanolamine, or

(ii) mixing a source of silica, a source of alumina, a source of alkali metal, water and a source of ammonium ions in the absence of an alcohol or an alkylene oxide in the molar proportions (expressed in the case of silica and alumina sources in terms of the equivalent moles of the oxide, in the case of the alkali metal source in terms of the equivalent moles of the hydroxide (MOH) and in the case of the source of ammonium ions in terms of free ammonia):

$SiO_2:Al_2O_3$ greater than 12:1
$MOH:Al_2O_3$ in the range from 1:1 to 20:1
$SiO_2:NH_3$ in the range from 1:1 to 200:1,
and
$H_2O:MOH$ in the range from 30:1 to 300:1

and maintaining the mixture at elevated temperature for a period such that crystallisation occurs, or

(iii) by maintaining an aqueous mixture containing the following compounds:

one or more compounds of an alkali and/or alkaline earth metal (M), one or more Al-compounds, one or more Si-compounds, one or more alcohols (ROH) and ammonia, in which mixture the various compounds are present in the following molar ratios, expressed, with the exception of the alcohols and ammonia, in moles of the oxides:

$SiO_2:Al_2O_3$ greater than or equal to 12,
$(M)_{2/n}O:SiO_2=0.01—1.0.$
$H_2O:(M)_{2/n}O=10—500$
$ROH:Al_2O_3=5—500,$
and
$ROH:NH_3$ greater than 2

wherein n is the valency of M at elevated temperature until the zeolite has been formed and subsequently separating the crystals of the zeolite from the mother liquor characterised in that the metal incorporated either by exchange or impregnation is aluminium.

It is understood that the aluminium incorporated into the aluminosilicate excludes aluminium forming part of the crystal lattice of the aluminosilicate and also excludes aluminium in the form of alumina which is part or all of a matrixing material sometimes used in conjunction with crystalline aluminosilicates.

The $C_2$ to $C_{12}$ aliphatic hydrocarbon feedstock may be a single hydrocarbon or a mixture of hydrocarbons. Furthermore the, or each, hydrocarbon in the feedstock may be saturated or unsaturated. Preferably the hydrocarbon is a $C_3$ to $C_8$, even more preferably a $C_3$ to $C_6$ hydrocarbon. $C_4$ hydrocarbon fractions containing isobutane and/or isobutene and $C_3$ hydrocarbon fractions containing propane and/or propylene are particularly preferred.

With regard to the catalyst, the crystalline aluminosilicate is produced by either:—

(i) crystallising a mixture containing a source of alumina, a source of silica, a source of alkali metal, water and a mono-, di- or tri-ethanolamine or propanolamine or their precursors as described, for example, in our copending European applications publication Nos 2899 and 2900, or (ii) by mixing a source of silica, a source of alumina, a source of alkali metal, water and a source of ammonium ions in the absence of an alkylene oxide in the molar proportions (expressed in the case of the silica and alumina sources in terms of the equivalent moles of the oxide, in the case of the alkali metal source in terms of the equivalent moles of the hydroxide (MOH) and in the case of the source of ammonium ions in terms of free ammonia):

$SiO_2:Al_2O_3$ greater than 12:1, preferably from 20:1 to 50:1
$MOH:Al_2O_3$ in the range from 1:1 to 20:1 preferably from 2:1 to 10:1
$SiO_2:NH_3$ in the range from 1:1 to 200:1, preferably from 20:1 to 100:1 and
$H_2O:MOH$ in the range from 30:1 to 300:1,
preferably from 30:1 to 100:1

and maintaining the mixture at elevated temperature for a period such that crystallisation occurs, as described, for example, in our copending European application publication No. 30811, or

(iii) by maintaining an aqueous mixture containing the following compounds:

one or more compounds of an alkali and/or alkaline earth metal (M), one or more Al-compounds, one or more Si-compounds, one or more alcohols (ROH) and ammonia, in

which mixture the various compounds are present in the following molar ratios, expressed, with the exception of the alcohols and ammonia, in moles of the oxides:

$SiO_2:Al_2O_3$ greater than or equal to 12,
$(M)_{2/n}O:SiO_2=0.01—1.0$
$H_2O:(M)_{2/n}O=10—500$
$ROH:Al_2O_3=5—500$,

and

$ROH:NH_3$ greater than 2

wherein n is the valency of M

at elevated temperature until the zeolite has been formed and subsequently separating the crystals of the zeolite from the mother liquor, as described for example in UK patent application publication No. 2018232A (Shell International Research).

The crystalline aluminosilicate is modified by incorporation therein of aluminium either by exchange or impregnation. With regard to exchange any of the conventional ion-exchange techniques may be employed. Suitably the crystalline aluminosilicate may first of all be exchanged with ammonium ions and subsequently exchanged with aluminium ions. The exchange with ammonium ions may suitably be effected with an aqueous solution of an ammonium salt, such as the chloride. The exchange with aluminium ions may suitably be effected with an aluminium salt such as the sulphate, nitrate, acetate or a halide, eg the chloride. After exchange the crystalline aluminosilicate modified by incorporation of exchanged aluminium may suitably be dried.

Alternatively the crystalline aluminosilicate may be modified by incorporation of aluminium therein by impregnation. Conventional impregnation techniques may be used to produce this form of the catalyst. The impregnation may suitably be conducted by preparing a solution, suitably an aqueous solution of an aluminium salt such as for example aluminium nitrate and adding the crystalline aluminosilicate to this aqueous solution accompanied by stirring to form a paste. The paste may thereafter be dried. It is believed that the aluminium is incorporated on the surface of the aluminosilicate, in the intracrystalline cavities and, inevitably to some extent, as exchanged cations.

Whichever method of catalyst preparation is used, the amount of aluminium present may suitably be in the range from 0.1 to 10%, preferably in the range from 0.5 to 7% by weight of the crystalline aluminosilicate present.

The catalyst may suitably be activated prior to contact with the aliphatic hydrocarbon. Activation may be carried out by heating the catalyst at a temperature in the range from 400 to 650°C, preferably in the range from 500 to 600°C. Activation may be conducted in an atmosphere of hydrogen, air or a gas which is substantially inert under the reaction conditions such as nitrogen. The catalyst may suitably be used in the form of a fixed or a fluidised bed.

The elevated temperature at which the aliphatic hydrocarbon is contacted with the catalyst may suitably be in the range 450°C to 700°C, preferably in the range 500° to 600°C.

The process is preferably operated continuously.

A substantially inert diluent such as nitrogen may be employed.

The invention will now be described with reference to the following Examples.

In the following examples reference will be made to Ludox Type AS40 silica sol which is a silica sol containing 40% wt/wt silica and having ammonium ions as the stabilising counter ions. The silica to ammonium ions (i.e. $SiO_2:NH_3$) is 80:1 molar.

Preparation of catalysts

Catalyst A

(i) Alumina, Laporte Type A (13.35 g) was dissolved in a solution of sodium hydroxide (26.25 g) in deionised water (187.5 ml) by warming. This solution was then added with stirring to Ludox silica sol, Grade AS40 (590 g) and 0.910 aqueous ammonia solution (9.0 ml, containing 25% wt/wt ammonia). The pH of the mixture was 13:1.

The mixture was placed in a stirred stainless steel autoclave and heated at 170°C for 60 hours. A crystalline aluminosilicate product was formed. The solid product was filtered and ion-exchanged by refluxing with one molar ammonium chloride solution (500 ml). This operation was repeated twice. The mixture was filtered and the solid washed with deionised water and dried at 120°C for 16 hours.

(ii) 20 g of the dry powder were mixed with Ludox silica sol, Grade A40 (15 g) and sufficient deionised water to form a thick paste. The whole was dried at 120°C for 16 hours and broken down to form 5 to 16 mesh (BSS) granules. The catalyst was activated by calcining in air at 500°C for 16 hours.

Catalyst B

15 g of the aluminosilicate prepared as described under Catalyst A(i) above were ion-exchanged by heating with a solution of aluminium nitrate nonahydrate (12.5 g) in deionised water (300 ml) with stirring for 1 hour. This operation was carried out three times. The mixture was filtered and the solid washed with deionised water (300 ml) and dried at 120°C for 16 hours.

10 g of the dry powder were mixed with Ludox silica sol, Grade AS40 (10 g) to form a paste. The whole was dried at 120°C for 16 hours and broken down to form 5 to 16 mesh (BSS) granules. The catalyst was activated by calcining in air at 500°C for 16 hours.

Catalyst C

(i) Sodium aluminate (12.4 g, 90% purity=0.07 mole alumina) was dissolved in a solution of sodium hydroxide (4.43 g, 0.111 mole) in deionised water (200 g, 11.11 mole). The solution was filtered and then added to diethanolamine 116.1 g, 1.106 mole at 40°C. Ludox colloidal silica (317.3 g, containing 40% silica, 2.115 mole) dissolved in deionised water (176.4 g, 9.8 mole) was then added with stirring and stirring was continued for a further 30 minutes. The mixture was transferred to a one litre autoclave and heated at 170°C for 7 days. The pressure recorded was 10 bars. A crystalline aluminosilicate product was formed. The solid product was filtered, washed with deionised water and then ion-exchanged by refluxing with one molar ammonium chloride solution (500 ml). This operation was carried out twice. The mixture was filtered and the solid washed with deionised water and dried at 120°C for 16 hours.

(ii) 20 g of the dry powder prepared as described above were mixed with Ludox silica sol, Grade AS40 (20 g, containing 40% wt silica) and sufficient deionised water to form a thick paste. The whole was dried at 120°C for 16 hours and broken down to form 5 to 16 mesh (BSS) granules. The catalyst was activated by calcining in air at 500°C for 16 hours.

Catalyst D

10 g of the aluminosilicate prepared as described under Catalyst C(i) above was ion-exchanged by refluxing with a solution of aluminium nitrate nonahydrate (10.75 g) in deionised water (60 ml) for 4 hours. The mixture was filtered and the solid washed with deionised water (250 ml) and dried at 120°C for 16 hours.

10 g of the dry powder was mixed with Ludox silica sol, Grade AS40 (10 g) to form a paste. The whole was dried at 120°C for 16 hours and broken down to form 5 to 16 mesh (BSS) granules. The catalyst was activated by calcining in air at 500°C for 16 hours.

Testing of catalysts

Example 1

The activity of Catalyst B was tested by passing a gaseous feed of a $C_3$ hydrocarbon mixture (78.1% propane, 19.1% propylene and 2.8% ethane) over the catalyst in a heated glass reactor. The conditions used and the results obtained are given in the following Tables 1A and 1B respectively.

Comparison Test 1

Example 1 was repeated except that Catalyst B was replaced by Catalyst A. The conditions used and the results obtained are given in the following Tables 1A and 1B respectively. This is not an example according to the invention because the crystalline aluminosilicate was not modified by incorporation of aluminium. It is included only for the purpose of comparison.

Example 2

The activity of Catalyst D was tested by passing a gaseous feed of propane over the catalyst in a heated glass reactor. The conditions used and the results obtained are given in Tables 2A and 2B.

Comparison Test 2

Example 2 was repeated except that Catalyst D was replaced by Catalyst C. The conditions used and the results obtained are given in Tables 2A and 2B respectively. This is not an example according to the invention because the crystalline aluminosilicate was not modified by incorporation of aluminium. It is included only for the purpose of comparison.

The results using Catalyst B illustrate that incorporating aluminium into the zeolite prepared by the method described in our copending European Application Publication No. 30811 produces a catalyst which is much more active for the dehydrocyclodimerisation of $C_3$ hydrocarbons into aromatics than the untreated zeolite.

The results using Catalyst D demonstrate that incorporating aluminium into the zeolite prepared by the method described in our copending European Application Publication No. 2900 produces a catalyst which is more active for the dehydrocyclodimerisation of $C_3$ hydrocarbons into aromatics than the untreated zeolite.

TABLE 1A
Conditions used

| Example | Catalyst | Hours on stream | Reaction temperature °C | Contact[1] time sec |
|---|---|---|---|---|
| Comp Test 1 | A | 2 | 550 | 17.9 |
| Example 1 | B | 2 | 550 | 16.2 |

TABLE 2A
Conditions used

| Example | Catalyst | Hours on stream | Reaction temperature °C | Contact[1] time sec |
|---|---|---|---|---|
| Comp Test 2 | C | 2 | 550 | 13.0 |
| Example 2 | D | 2 | 550 | 16.0 |

With reference to the above Tables 1A and 2A:

$$^{1.}\ \text{Contact time} = \frac{\text{Volume of catalyst in mls}}{\text{Total volume of gas (in mls/sec at NTP)}}$$

TABLE 1B
Results obtained

| Example | Catalyst | $C_3$ Conversion[2] % | Molar Yields[3] % | | | | Selectivity[4] to aromatics | Composition of Aromatics[5] % | | |
|---|---|---|---|---|---|---|---|---|---|---|
| | | | $CH_4$ | $C_2H_4$ | $C_2H_6$ | Aromatics | | Benzene | Toluene | Xylene |
| Comp Test 1 | A | 58 | 15 | 2 | 15 | 26 | 45 | 15 | 28 | 18 |
| Example 1 | B | 89 | 22 | 3 | 12 | 52 | 58 | 33 | 35 | 11 |

With reference to the above Table

2. $C_3$ Conversion $= \frac{\text{Moles of } C_3 \text{ hydrocarbon consumed}}{\text{Moles of } C_3 \text{ hydrocarbon fed}} \times 100$

3. Molar Yield $= \frac{\text{Moles of } C_3 \text{ hydrocarbon converted to particular product}}{\text{Moles of } C_3 \text{ hydrocarbon fed}}$

4. Selectivity $= \frac{\text{Moles of } C_3 \text{ hydrocarbon converted to aromatics}}{\text{Moles of } C_3 \text{ hydrocarbon consumed}} \times 100$

5. Remainder poly alkyl aromatics.

TABLE 2B
Results obtained

| Example | Catalyst | $C_3$ conversion[2] % | Molar Yields[3] % | | | | Selectivity[4] to aromatics | Composition of aromatics[5] % | | |
|---|---|---|---|---|---|---|---|---|---|---|
| | | | $CH_4$ | $C_2H_4$ | $C_2H_6$ | Aromatics | | Benzene | Toluene | Xylene |
| Comp Test 2 | C | 67 | 18 | 5 | 12 | 32 | 48 | 28 | 46 | 21 |
| Example 2 | D | 83 | 18 | 3 | 12 | 50 | 60 | 29 | 41 | 16 |

With reference to the above Table

2. $C_3$ Conversion $= \frac{\text{Moles of } C_3 \text{ hydrocarbon consumed}}{\text{Moles of } C_3 \text{ hydrocarbon fed}} \times 100$

3. Molar Yield $= \frac{\text{Moles of } C_3 \text{ hydrocarbon converted to particular product}}{\text{Moles of } C_3 \text{ hydrocarbon fed}}$

4. Selectivity $= \frac{\text{Moles of } C_3 \text{ hydrocarbon converted to aromatics}}{\text{Moles of } C_3 \text{ hydrocarbon consumed}} \times 100$

5. Remainder poly alkyl aromatics.

## Claims

1. A process for the production of aromatic hydrocarbons by contacting at an elevated temperature and in the vapour phase a $C_2$ to $C_{12}$ aliphatic hydrocarbon feedstock with a catalyst comprising a crystalline aluminosilicate having a silica to alumina molar ratio greater than 12:1 which has been modified by incorporation therein of a metal either by exchange or impregnation and which is produced by either

(i) crystallising a mixture containing a source of alumina, a source of silica, a source of alkali metal, water and a mono-, di- or tri-ethanolamine or propanolamine, or

(ii) mixing a source of silica, a source of alumina, a source of alkali metal, water and a source of ammonium ions in the absence of an alcohol or an alkylene oxide in the molar proportions (expressed in the case of silica and alumina sources in terms of the equivalent moles of the oxide, in the case of the alkali metal source in terms of the equivalent moles of the hydroxide (MOH) and in the case of the source of ammonium ions in the terms of free ammonia):

$SiO_2:Al_2O_3$ greater than 12:1
$MOH:Al_2O_3$ in the range from 1:1 to 20:1
$SiO_2:NH_3$ in the range from 1:1 to 200:1,
and
$H_2O:MOH$ in the range from 30:1 to 300:1

and maintaining the mixture at elevated temperature for a period such that crystallisation occurs, or

(iii) by maintaining an aqueous mixture containing the following compounds:

one or more compounds of an alkali and/or alkaline earth metal (M), one or more Al-compounds, one or more Si-compounds, one or more alcohols (ROH) and ammonia, in which mixture the various compounds are present in the following molar ratios, expressed, with the exception of the alcohols and ammonia, in moles of the oxides:

$SiO_2:Al_2O_3$ greater than or equal to 12,
$(M)_{2/n}O:SiO_2=0.01—1.0$
$H_2O:(M)_{2/n}O=10—500$
$ROH:Al_2O_3=5—500$,
and
$ROH:NH_3$ greater than 2

wherein n is the valency of M

at elevated temperature until the zeolite has been formed and subsequently separating the crystals of the zeolite from the mother liquor characterized in that the metal incorporated either by exchange or impregnation is aluminium.

2. A process according to claim 1 wherein the feedstock is a $C_3$ to $C_6$ hydrocarbon.

3. A process according to either claim 1 or claim 2 wherein the feedstock is a $C_4$ hydrocarbon fraction containing isobutane and/or isobutene.

4. A process according to either claim 1 or claim 2 wherein the feedstock is a $C_3$ hydrocarbon fraction containing propane and/or propylene.

5. A process according to any one of the preceding claims wherein the crystalline aluminosilicate is produced by mixing a source of silica, a source of alumina, a source of alkali metal, water and a source of ammonium ions in the molar proportions:

$SiO_2:Al_2O_3$ in the range from 20:1 to 50:1,
$MOH:Al_2O_3$ in the range from 2:1 to 10:1,
$SiO_2:NH_3$ in the range from 20:1 to 100:1,
and
$H_2O:MOH$ in the range from 30:1 to 100:1.

6. A process according to any one of the preceding claims wherein the catalyst is activated by heating at a temperature in the range from 400 to 650°C prior to contact with the aliphatic hydrocarbon.

7. A process according to any one of the preceding claims wherein the amount of aluminium incorporated is in the range from 0.1 to 10% by weight of the crystalline aluminosilicate.

8. A process according to any one of the preceding claims wherein the aliphatic hydrocarbon is contacted with the catalyst at a temperature in the range 450 to 700°C.

9. A process according to any one of the preceding claims when operated continuously.

## Patentansprüche

1. Verfahren zur Herstellung von aromatischen Kohlenwasserstoffen durch Kontaktieren bei erhöhter Temperatur und in der Dampfphase eines $C_2$—$C_{12}$-aliphatischen Kohlenwasserstoff-Ausgangsmaterials mit einem Katalysator bestehend aus einem kristallinen Alumosilikat mit einem Siliciumdioxid zu Aluminiumoxid Molar-Verhältnis größer als 12:1, der modifiziert worden ist durch Einbau eines Metalls entweder durch Austausch oder durch Imprägnierung und der hergestellt worden ist durch enweder

(i) Kristallisierung einer Mischung enthaltend eine Quelle von Aluminiumoxid, eine Quelle von Kieselsäure, eine Quelle von Alkalimetall, Wasser und ein Mono-, Di- oder Triethanolamin oder -propanolamin, oder

(ii) Mischen einer Quelle von Kieselsäure, einer Quelle von Aluminiumoxid, einer Quelle von Alkalimetall, Wasser und einer Quelle von Ammoniumionen in Abwesenheit eines Alkohols oder eines Alkylenoxids in molaren Verhältnissen (ausgedrückt im Fall von Kieselsäure- und Aluminiumoxidquellen in Form von äquivalenten Molen des Oxids, im Falle der Alkalimetall-Quelle in Form von äquivalenten Molen des Hydroxids (MOH) und im Falle der Ammoniumionen-Quelle in Form von freiem Ammoniak):

$SiO_2:Al_2O_3$ größer als 12:1
$MOH:Al_2O_3$ im Bereich von 1:1 bis 20:1
$SiO_2:NH_3$ im Bereich von 1:1 bis 200:1,
und
$H_2:MOH$ im Bereich von 30:1 bis 300:1

und Halten der Mischung bei erhöhter Temperatur für eine Zeitspanne die ausreichend ist, daß Kristallisation eintritt; oder

(iii) Halten einer wässrigen Mischung enthaltend folgende Bestandteile: eine oder mehrere Verbindungen eines Alkali- und/oder Erdalkalimetalls (M), eine oder mehrere Al-Verbindungen, eine oder mehrere Si-Verbindungen, einen oder mehrere Alkohole (ROH) und Ammoniak, in welcher Mischung die verschiedenen Verbindungen anwesend sind in den folgenden molaren Verhältnissen, ausgedrückt, mit Ausnahme der Alkohole und des Ammoniaks, in Molen der Oxide:

$SiO_2:Al_2O_3$ größer also oder gleich 12,
$(M)_{2/n}O:SiO_2$=0,01—1,0
$H_2O:(M)_{2/n}O$=10—500
$ROH:Al_2O_3$=5—500,
und
$ROH:NH_3$ größer als 2

worin n die Wertigkeit von M bedeutet, bei erhöhter Temperatur bis der Zeolit gebildet ist und anschließende Trennung der Zeolitkristalle von der Mutterlauge, dadurch gekennzeichnet, daß das Metall welches durch Austausch oder Imprägnierung eingebaut wird, Aluminium ist.

2. Verfahren gemäß Anspruch 1, worin das Ausgangsmaterial ein $C_3$—$C_6$-Kohlenwasserstoff ist.

3. Verfahren gemäß Anspruch 1 oder 2, worin das Ausgangsmaterial eine $C_4$-Kohlenwasserstoffraktion enthaltend Isobutan and/oder Isobuten ist.

4. Verfahren gemäß Anspruch 1 oder 2, worin das Ausgangsmaterial eine $C_3$-Kohlenwasserstoffraktion enthaltend Propan und/oder Propylen ist.

5. Verfahren gemäß einem der vorgehenden Ansprüche, worin das kristalline Alumosilikat hergestellt wird durch Vermischen einer Quelle von Kieselsäure, einer Quelle von Aluminiumoxid, einer Quelle von Alkalimetall, Wasser und einer Quelle von Ammoniumionen in molaren Verhältnissen:

$SiO_2:Al_2O_3$ im Bereich von 20:1 bis 50:1,
$MOH:Al_2O_3$ im Bereich von 2:1 bis 10:1,
$SiO_2:NH_3$ im Bereich von 20:1 bis 100:1,
und
$H_2O:MOH$ im Bereich von 30:1 bis 100:1.

6. Verfahren gemäß einem der vorgehenden Ansprüche, worin der Katalysator aktiviert wird durch Erhitzen auf eine Temperatur im Bereich von 400 bis 650°C bevor er mit dem aliphatischen Kohlenwasserstoff in Berührung gebracht wird.

7. Verfahren gemäß einem der vorgehenden Ansprüche, worin die Menge an eingebautem Aluminium im Bereich von 0,1 bis 10 Gew.-% des kristallinen Alumosilikats liegt.

8. Verfahren gemäß einem der vorgehenden Ansprüche, worin der aliphatische Kohlenwasserstoff mit dem Katalysator bei einer Temperatur im Bereich von 450 bis 700°C in Berührung gebracht wird.

9. Verfahren gemäß einem der vorgehenden Ansprüche wenn dieses kontinuierlich betrieben wird.

**Revendications**

1. Procédé de préparation d'hydrocarbures aromatiques par mise en contact, à température élevée et en phase vapeur, d'une charge d'hydrocarbures aliphatiques en $C_2$ à $C_{12}$ d'alimentation avec un catalyseur comprenant un aluminosilicate cristallin présentant, entre la silice et l'alumine, un rapport molaire supérieur à 12:1, qui a été modifié par incorporation d'un métal par échange ou imprégnation et qui est produit par (i) cristallisation d'un mélange contenant une source d'alumine, une source de silice, une source de métal alcalin, de l'eau et une mono- di- ou tri- éthanolamine ou propanolamine, ou (ii) mélangeage d'une source de silice, d'une source d'alumine, d'une source de métal alcalin, d'eau et d'une source d'ions ammonium en l'absence d'un alcool ou d'un oxyde d'alkylène, selon les proportions molaires (exprimées dans le cas des sources de silice et d'alumine en termes de moles d'équivalent de l'oxyde, dans le cas de la source de métal alcalin en termes de moles d'équivalent de l'hydroxyde (MOH) et dans le cas de la source d'ions ammonium en termes d'ammoniac libre):

$SiO_2:Al_2O_3$ supérieur à 12:1
$MOH:Al_2O_3$ compris entre 1:1 et 20:1
$SiO_2:NH_3$ compris entre 1:1 et 200:1,
et
$H_2O:MOH$ compris entre 30:1 et 300:1

et maintien du mélange à température élevée pendant une période telle qu'une cristallisation se produise, ou (iii) par maintien d'un mélange aqueux contenant les composés suivants:

un ou plusieurs composés d'un métal alcalin et/ou alcalinoterreux (M), un ou plusieurs composés de Al, un ou plusieurs composés de Si, un ou plusieurs alcools (ROH) et de l'ammoniac, mélange dans lequel les divers composés sont présents selon les rapports molaires suivants, exprimés, à l'exception des

alcools et de l'ammoniac, en moles des oxydes:

$SiO_2:Al_2O_3$ supérieur ou égal à 12,
$(M)_{2/n}O:SiO_2$=0,01 à 1,0
$H_2O:(M)_{2/n}O$=10 à 500
$ROH:Al_2O_3$=5 à 500,

et

$ROH:NH_3$ supérieur à 2,

où n est la valence de M,

à température élevée jusqu'à formation de la zéolite, puis séparation des cristaux de la zéolite d'avec la liqueur mère, procédé caractérisé en ce que le métal incorporé par échange ou imprégnation est l'aluminium.

2. Procédé selon la revendication 1, dans lequel la charge d'alimentation est un hydrocarbure en $C_3$ à $C_6$.

3. Procédé selon la revendication 1 ou la revendication 2, dans lequel la charge d'alimentation est une fraction d'hydrocarbure(s) en $C_4$ contenant de l'isobutane et/ou de l'isobutène.

4. Procédé selon la revendication 1 ou la revendication 2, dans lequel la charge d'alimentation est une fraction d'hydrocarbure(s) en $C_3$ contenant du propane et/ou du propylène.

5. Procédé selon l'une quelconque des revendications précédentes, dans lequel l'aluminosilicate cristallin est préparé par mélangeage d'une source de silice, d'une source d'alumine, d'une source de métal alcalin, d'eau et d'une source d'ions ammonium selon les proportions molaires:

$SiO_2:Al_2O_3$ compris entre 20:1 et 50:1,
$MOH:Al_2O_3$ compris entre 2:1 et 10:1,
$SiO_2:NH_3$ compris entre 20:1 et 100:1,

et

$H_2O:MOH$ compris entre 30:1 et 100:1.

6. Procédé selon l'une quelconque des revendications précédentes, dans lequel le catalyseur est activé par chauffage à une température comprise entre 400 et 650°C avant sont contact avec l'hydrocarbure aliphatique.

7. Procédé selon l'une quelconque des revendications précédentes, dans lequel la quantité d'aluminium incorporée se situe entre 0,1 et 10% du poids de l'aluminosilicate cristallin.

8. Procédé selon l'une quelconque des revendications précédentes, dans lequel on met l'hydrocarbure aliphatique au contact du catalyseur à une température comprise entre 450 et 700°C.

9. Procédé selon l'une quelconque des revendications précédentes, quand il est mis en oeuvre de façon continue.